# EUROPEAN PATENT SPECIFICATION

(11) **EP 1 313 499 B1**
(45) Date of publication and mention of the grant of the patent: **07.04.2010**
(21) Application number: 01968180.8
(22) Date of filing: 28.08.2001
(51) Int. Cl.: A61K 38/30, A61P 25/00

(54) **METHOD FOR TREATING THE CENTRAL NERVOUS SYSTEM BY ADMINISTRATION OF IGF STRUCTURAL ANALOGS**
METHODE ZUR BEHANDLUNG DES ZENTRALNERVENSYSTEMS DURCH APPLIKATION VON STRUKTURANALOGA VON IGF
METHODE DE TRAITEMENT DU SYSTEME NERVEUX CENTRAL PAR ADMINISTRATION D'ANALOGUES STRUCTURAUX D'IGF

(30) Priority: 29.08.2000 US 228633 P
(43) Date of publication of application: 28.05.2003
(73) Proprietor: Aurogen Incorporated, Fort Collins, Colorado 80522 (US)
(72) Inventor: ISHII, Douglas, N., LaPorte, CO 80535 (US)
(74) Representative: Clarkson, Paul Magnus
(86) International application number: PCT/US2001/026750
(87) International publication number: WO 2002/017951

(56) References cited:
- WO-A-91/12018
- US-A- 4 876 242
- US-A- 5 420 111
- US-A- 5 473 054
- LODDICK S A ET AL: "Displacement of insulin-like growth factors from their binding proteins a s a potential treatment for stroke" PROCEEDINGS OF THE NATIONAL ACADEMY OF SCIENCES OF USA, NATIONAL ACADEMY OF SCIENCE. WASHINGTON, US, no. 95, 1 February 1998 (1998-02-01), pages 1894-1898, XP002074895 ISSN: 0027-8424
- DATABASE BIOSIS [Online] BIOSCIENCES INFORMATION SERVICE, PHILADELPHIA, PA, US; 1991 LEMMEY A B ET AL: "IGF-I AND THE TRUNCATED ANALOGUE DES-1-3IGF-I ENHANCE GROWTH IN RATS AFTER GUT RESECTION" Database accession no. PREV199191105965 XP002274574 & AMERICAN JOURNAL OF PHYSIOLOGY, vol. 260, no. 2 PART 1, 1991, pages E213-E219, ISSN: 0002-9513

## Description

### SUMMARY OF THE INVENTION

This invention is directed to the use in the manufacture of a medicament for treating the central nervous system by the nonintracranial and nonintravertebral column administration of one or more IGF structural analogs. More particularly, the invention is directed to treating disorders or diseases of the brain or spinal cord by the nonintracranial or nonintravertebral column administration of one or more IGF structural analogs.

Loddick et al. in Proc. Nat Acad Sc USA, 95, February 1998, pp 1894-1898 discloses the use of structural IGF-1 analogs for the treatment of stroke and neurodegenerative diseases after intracerebroventricular administration. WO91/12018 discloses the use of IGF-I structural analogs for the treatment of intesttinal diseases after subcutanous injection. Lemmay et al. in Bioscience information service, 1991, discloses the anabolic effect of structural IGF-1 analogs applied by osmotic pumps after surgical trauma by gut resection. US-A-4876242 discloses the manufacture of synthetic IGF-I analogs and its stimulation of some growth processes. US-A-5420111 discloses the use of structural IGF-I or -II analogs for the treatment of fetal growth retardation. US-A-5473054 discloses the treatment of restenosis of coronary arteries, neoplasia and brain metastases by structural analogs of IGF-I.

### DESCRIPTION OF DRAWINGS

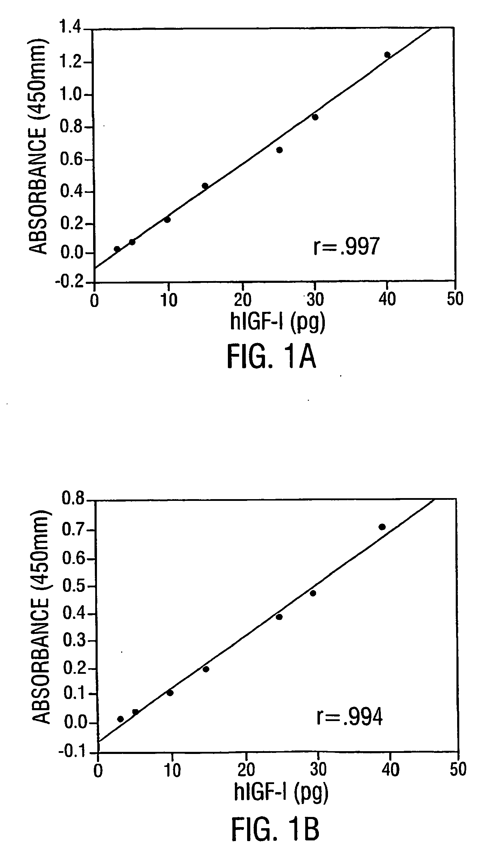

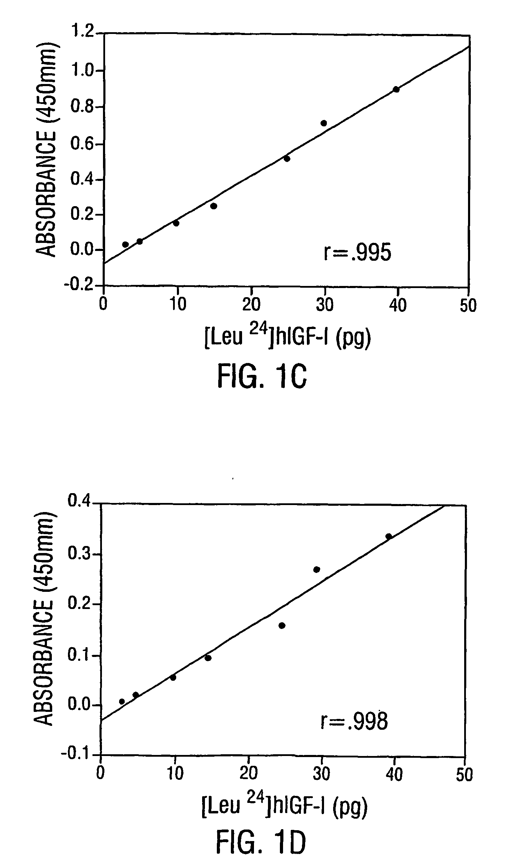

FIG. 1. Concentration-dependent detection of (A) hIGF-I, (B) Des(1-3)hIGF-I, (C) [Leu²⁴]hIGF-I and (D) [Leu⁶⁰]hIGF-I by ELISA. Samples were assayed in triplicate at each concentration. The coefficient of correlation, r, was determined by linear regression using a computer software program.

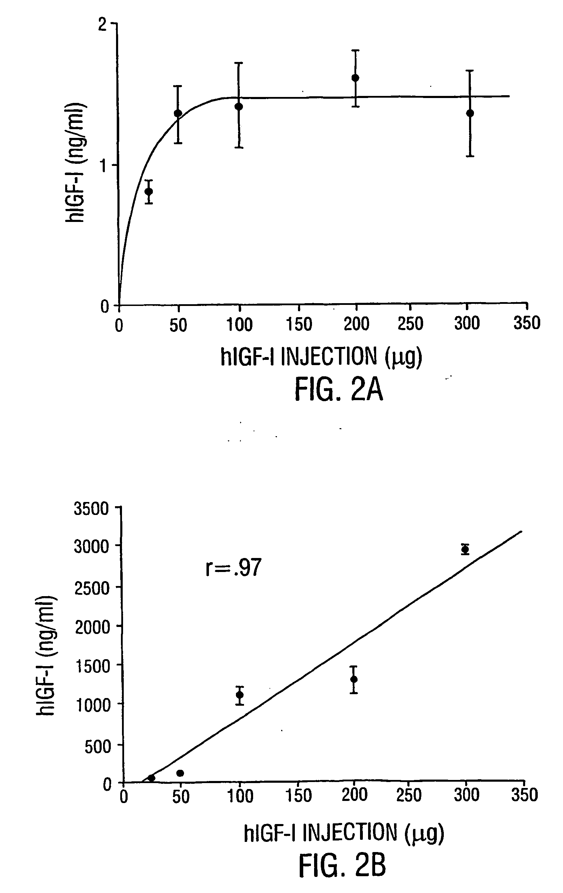

FIG. 2. Dose-dependent distribution of immunoreactive hIGF-I in CSF and plasma following subcutaneous injections in adult rats. Plasma and CSF were withdrawn for ELISA 90 min after a single bolus subcutaneous injection of the indicated dose of hIGF-I, and each sample was assayed in triplicate. Group means ± SEM are shown (n = 3 rats per dose). Part A, CSF hIGF-I. Part B, plasma hIGF-I. The data were plotted using linear regression, r = 0.97.

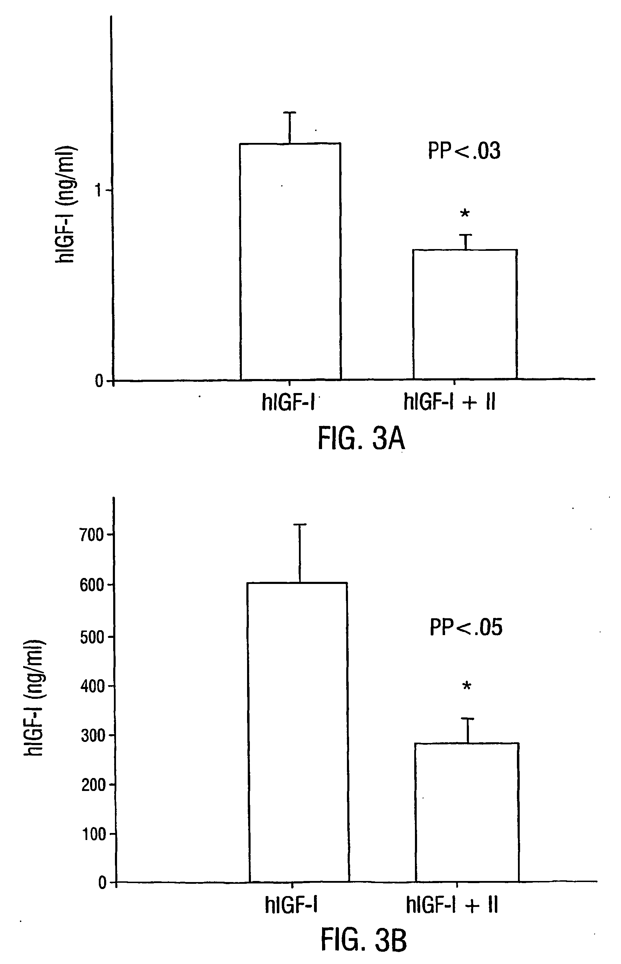

FIG. 3. Effect of simultaneous administration of hIGF-II on hIGF-I uptake into CSF. Rats were injected subcutaneously with 150 µg hIGF-I alone (n = 8) or the combination of 150 µg hIGF-I and 400 µg hIGF-II (n = 6). Plasma and CSF were withdrawn 90 min later for assay. Values are means ± SEM. The group means were compared using a t-test. *P<0.02.

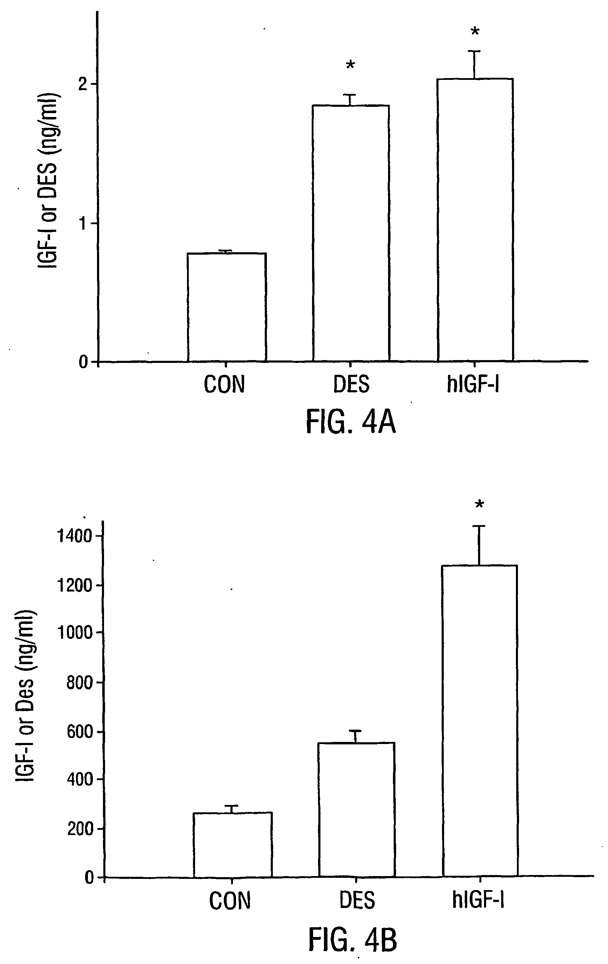

FIG. 4. Comparative distribution in CSF and plasma following administration of Des(1-3)hIGF-I (n = 4), hIGF-I (n = 3), or vehicle (n = 2). Equivalent amounts (200 µg per rat) of Des(1-3)hIGF-I or hIGF-I were injected subcutaneously, and plasma and CSF were withdrawn for assay 90 min later. Group means were compared using Newman-Keuhl's posthoc test. *P<0.002 and 0.003 for Des and hIGF-I, respectively, vs. control in CSF. *P<0.002 for hIGF-I vs. control in plasma.

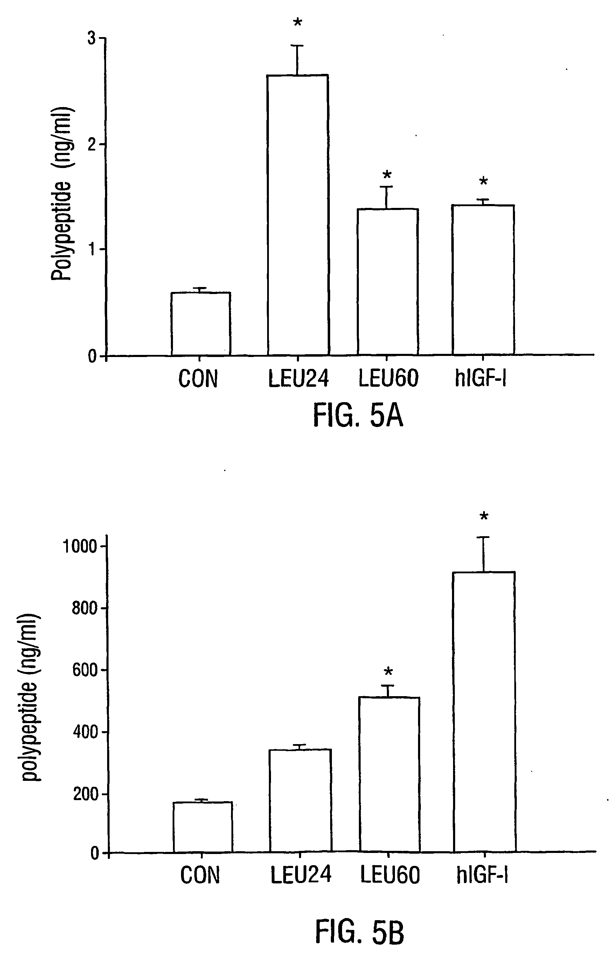

FIG. 5. Uptake of [Leu²⁴]hIGF-I and [Leu⁶⁰]hIGF-I into CSF. [Leu²⁴]hIGF-I (200 µg per rat; n = 3 rats), [Leu⁶⁰]hIGF-I (100 µg per rat; n = 4) or hIGF-I (200 µg per rat; n = 3) or vehicle (n = 9) were injected subcutaneously, and 90 min later plasma and CSF were withdrawn for assay. Part A, CSF; Part B, plasma. Differences between group means were detected using Newman-Keuhl's posthoc test. *P<0.002 for hIGF-I vs. [Leu²⁴]hIGF-I in CSF. *P<0.0004 for [Leu²⁴] and [Leu⁶⁰] vs. control and 0.0007 for hIGF-I vs. control in CSF. In plasma, *P<0.0002 and 0.0005 for [Leu²⁴] and [Leu⁶⁰], respectively, vs. hIGF-I. *P<0.0005 and 0.0002 for [Leu⁶⁰] and hIGF-I, respectively, vs. control.

### DETAILED DESCRIPION OF THE INVENTION

This invention is directed to the use in the manufacture of a medicament for treating the central nervous system by the nonintracranial and nonintravertebral column administration of one or more IGF structural analogs. More particularly, the invention is directed to treating disorders or diseases of the brain or spinal cord by the nonintracranial or nonintravertebral column administration of one or more IGF structural analogs. For purposes of this invention, "IGF structural analogs" are defined as molecules having substantial sequence homology to naturally occurring insulin-like growth factors (IGFs), including human and animal (including but not limited to cow, pig, dog, sheep, horse, deer, goat, rat, mouse and chicken) IGF-I and IGF-II. More preferably, the IGF structural analogs have amino acid sequences of IGF molecules that have been modified by deletions, substitutions and/or additions of fewer than 15 amino acids.

According to the invention, the preferred route of administration of the IGF structural analog is from a site outside of the blood-brain-barrier (BBB), blood-central nervous system-barrier (B-CNS-B) and blood-spinal cord-barrier (B-SC-B). Any of the common routes of administration known to the pharmaceutical sciences may be used that can deliver IGF structural analogs into the circulation, including but not limited to percutaneous, intradermal, subcutaneous, intravenous, intramuscular, intraarterial, intraperitoneal, parenteral, buccal, sublingual, rectal, oral, nasal, by inhalation, from a subcutaneous implanted pump or matrix, or from a plasmid construct containing an IGF structural analog gene that is administered at a site outside of the BBB, B-CNS-B and B-SC-B. For example, the nasal cavity and lung are richly vascularized, and IGF structural analogs administered into the nasal cavity or by inhalation may be rapidly taken up by the local microvasculature, resulting in IGF analogs being taken up into cerebrospinal fluid (CSF) across the BBB or B-CNS-B. This invention is not limited to a particular route of administration, other than that the administration is from a site outside of the BBB, B-CNS-B and B-SC-B.

In a preferred embodiment, an IGF structural analog may be administered alone or in combination with other IGF analogs. The IGF analog may also be combined with one or more excipients, coloring agents, salts, solvents, carriers, stabilizers, and other ingredients that may be used in formulations and are known to the pharmaceutical sciences. In a further preferred embodiment, the IGF structural analog is administered in an amount from about 0.01 µg/kg/day up to about 4 mg/kg/day.

In a preferred embodiment, the invention is directed to treating disorders or diseases of the postbirth brain or spinal cord, such as Alzheimer's Disease, Parkinson's Disease, AIDS-related dementia, senile dementia, stroke, trauma, cortical-basal ganglionic syndromes, progressive dementia, familial dementia with spastic paraparesis, progressive supranuclear palsy, multiple sclerosis, hepatic encephalopathy, Pick's Disease, Huntington's Disease, diffuse cerebral sclerosis of Schilder, acute necrotizing hemorrhagic encephalomyelitis, brain tumors and the like. This invention does not include amyotrophic lateral sclerosis.

IGF structural analogs that may be used in the present invention include but are not limited to des(1-3)IGF-I, which is an IGF-I analog lacking the N-terminal tripeptide; [Arg3]IGF-I, which is an IGF-I analog in which Arg is substituted for Glu at position 3; [Leu24]IGF-I, which is an IGF-I analog in which Leu is substituted for Thr at position 24; [Leu60]IGF-I, which is a mutant IGF-I with Leu substituted for Tyr at position 60; Long R3IGF-I, which is a mutant IGF-I with Arg substituted for Glu at position 3 as well as a 13 amino acid extension at the N-terminus; des(1-6)IGF-II, which is an IGF-II analog lacking the N-terminal hexapeptide; [Glyl]IGF-II, which is an IGF-II mutant with Gly substituted for Ala at position 1; [Arg6]IGF-II, which is an IGF-II mutant with an Arg substituted for Glu at position 6; and [Leu27]IGF-II, which is an IGF-II mutant with Leu substituted for Tyr at position 27. These IGF structural analogs are commercially available, for example, from GroPep, Pty. Ltd. (Australia). It is appreciated that in the art it is possible to produce various additional IGF structural analogs.

The IGF structural analogs used in the present invention have biological activity. For example, it is known that des(1-3)IGF-I.administered into the eye can enhance the growth of transplanted spinal cord, cerebral cortex and parietal cortex in the eye. It can increase choline acetyltransferase activity in cultured spinal cord and enhance growth of cultured olfactory bulb cells. [Arg3]IGF-I, long R³IGF-I, [Leu24]IGF-I, [Leu60]IGF-I, des(1-6)IGF-II, [Glyl]IGF-II, [Arg6]IGF-II, and [Leu27]IGF-II can bind to type I IGF receptors, type II IGF receptors, or IGF binding proteins and alter protein synthesis in cells. Thus, IGF structural analogs that cross the BBB, B-CNS-B or B-SC-B may be used for the purposes of this invention.

The treatment of the brain and spinal cord is more complicated than the treatment of the peripheral nervous system because the B-CNS-B, B-SC-B and BBB pose an obstacle to the delivery of pharmaceutical agents, particularly proteins and peptides, to the central nervous system. These barriers are widely believed to prevent the uptake and penetration of proteins and peptides, such as IGFs, and these concerns would apply equally well to IGF structural analogs. Applicant has previously shown that IGF-I or IGF-II can cross from the blood into the CSF and normalize brain biochemistry in disease, prevent loss of axons in the spinal cord, and prevent functional damage to the central nervous system. Therefore, based on subsequent research, it is expected that IGF structural analogs can likewise cross from the blood into the cerebral spinal fluid (CSF) and may prevent damage, disease or disorder in the central nervous system.

The following examples show that IGF structural analogs can enter the CSF from the circulation. Consequently, IGF structural analogs may effect changes in or treat the central nervous system. The examples show that there is a carrier that takes IGFs up from the circulation into CSF, and the properties of this carrier differ from known IGF binding proteins and IGF receptors, such as type I IGF receptor or type II IGF receptor. The IGF analogs in the examples that are taken up into CSF includes des(I-3)IGF-I, [Leu24]IGF-I and [Leu60]IGF-I. Furthermore, IGF-II reduces IGF-I uptake into CSF, and this is consistent with competition for uptake by a common IGF carrier. The invention in its broader aspects is not limited to the specific details or representative examples described. Therefore, based on subsequent research, it is expected that IGF structural analogs that are taken up into CSF by this carrier may be used for the purposes of this invention. Those IGF structural analogs that are taken up into CSF may serve as agonists or antagonists. Antagonists may be useful for inhibiting the growth of brain tumors that may be IGF-dependent, for example. Agonists may be useful for treating various brain diseases and disorders such as Parkinson's Disease, Alzheimer's Disease, multiple sclerosis, stroke, trauma, senile dementia, and the like.

In the examples, IGF structural analogs were injected subcutaneously into rats. Ninety minutes later plasma and CSF were withdrawn and analyzed by an ELISA (Table I).

**TABLE 1. Selective detection of hIGF-I and its analogs by ELISA**

| Sample | OD (450) Mean ± SEM | P Value |
|---|---|---|
| Blank | 0.0 ± 0 | |
| Human IGI-I (150 pg) | 0.568 ± .113 | 0.001 |
| Des (1-3) hIGF-1 (150 pg) | 0.276 ± .047 | 0.001 |
| Leu 24 hIGF-I (150 pg) | 0.661 ± .072 | 0.001 |
| Human IGF-II (150 pg) | 0.004 ± .017 | 0.959 |
| Insulin (150 pg) | 0.016 ± .006 | 0.903 |
| Rat CSF (extracted) | 0.018 ± .034 | 0.971 |
| Rat Plasma (extracted) | 0.051 ± .037 | 0.818 |

| | | |
|---|---|---|
| hIGF-I and other proteins were subjected to the ELISA shown in FIG. 1. Untreated rat CSF and plasma were tested at the same volumes assayed throughout these experiments. Note that rat IGF-I, IGF-II, insulin and IGFBP in CSF and plasma do not interfere in the ELISA. Values are means ± SEM of four replicate measurements. | | |

The ELISA detected human IGF-I and IGF structural analogs. However, the ELISA did not detect IGF-II or insulin. Furthermore, nothing in untreated rat CSF or plasma interfered with the ELISA, showing that this test was specific for human IGF-I and IGF structural analogs. In other words, endogenous rat IGF-I, rat IGF-II, rat insulin and other rat substances in CSF or plasma did not interfere in the ELISA. Fig. 1 shows standard ELISA curves for different concentrations of human IGF-I, des(1-3)IGF-I, [Leu24]IGF-I and [Leu60]IGF-I.

Adult rats were injected subcutaneously with various doses of human IGF-I. Fig 2 shows that IGF-I in plasma increased linearly with dose. However, IGF-I uptake into CSF saturated with increasing dose, showing that uptake was via an IGF uptake carrier. Fig. 3 shows that IGF-II competed with IGF-I for uptake into CSF.

### EXAMPLES

Example 1. Des(1-3)IGF-I is missing the first 3 amino acids from the N-terminus resulting in at least 25-fold reduced affinity for IGF binding protein-3 (IGFBP-3), IGFBP-4 and IGFBP-5. Binding to IGFBP-1 is reduced as well. Des(1-3)IGF-I binds to the type I IGF receptor, and has enhanced biological activity on neurons. It is more potent due to reduced binding to IGFBP. Fig. 4 shows that des(1-3)IGF-I administered subcutaneously is taken up into cerebrospinal fluid in adult rats. Therefore, binding of IGF and mutant IGFs to IGFBP-1, -3, -4 and -5 is not required for uptake into CSF, and the IGF uptake carrier molecule does not have characteristics of IGFBP-1, -3, -4 or -5.

Example 2. Leu is substituted for Thr at position 24 in [Leu24]IGF-I. Following subcutaneous injection of [Leu24]IGF-I into adult rats, it was readily detected in cerebrospinal fluid (Fig. 5). This, together with Examples 1 and 3, shows that IGF structural analogs with various deletions or substitutions can be taken up into CSF from the circulation.

Example 3. Leu has been substituted for Tyr at position 60 in [Leu60]IGF-I, which has a 20-fold reduced affinity for the type I IGF receptor. Following subcutaneous injection of [Leu60]IGF-I into adult rats, it was readily detected in cerebrospinal fluid (Fig. 5). This shows that binding to the type I IGF receptor is not necessary for uptake of IGFs, and the IGF carrier molecule does not have characteristics of the type I IGF receptor.

Des(1-3)IGF-I and IGF-I do not bind appreciably to the type II IGF receptor, yet both of these ligands are taken up into CSF following subcutaneous administration. Thus, binding to the type II IGF receptor is not required for uptake of IGFs into CSF, and the IGF carrier molecule does not have characteristics of the type II IGF receptor.

Uptake of insulin-like growth factors (IGFs) from the circulation into cerebrospinal fluid (CSF) is consistent with a transport carrier protein. This carrier protein does *not* have the same properties as the type I or type II IGF receptors, or IGF binding proteins. Consequently, the carrier has properties unlike that of previously characterized IGF binding molecules.

Therefore, IGF structural analogs are shown to enter the CSF from across the BBB, B-CSF-B and/or B-SC-B in a mammal. This invention has the advantage that mutant IGFs and IGF analogs may be administered from outside of the BBB, B-CSF-B and B-SC-B, and it would not be necessary to use invasive and riskier methods of administration such as intracranial or intrathecal. The risk and cost of surgery and risk of CNS infection may be circumvented by the invention.

## Claims

1. Use of an IGF structural analog in the preparation of a medicament for treating the central nervous system,
wherein the medicament is for treating or preventing neuronal damage in the central nervous system, except where the disease is amyotropic lateral sclerosis,
wherein the medicament is for nonintracranial and nonintravertebral column administration.

2. The use of claim 9, in which the damage is due to a disorder or disease in the central nervous system.

3. The use of claim 1 or 2, wherein the damage is due to a tumor or cancer.

4. The use of any of claims 1 to 3, wherein the neuronal damage to the central nervous system is due to a disorder or disease in the post-birth brain or spinal cord.

5. The use of claim 4, wherein the neuronal damage in the brain is due to hepatic encephalopathy, Pick's Disease, Huntington's Disease, diffuse cerebral sclerosis of Schilder, Alzheimer's Disease, Parkinson's Disease, AIDS-related dementia, senile dementia, stroke, trauma, cortical-basal ganglionic syndromes, progressive dementia, familial dementia with spastic paraparesis, progressive supranuclear palsy, multiple sclerosis, or acute necrotizing hemorrhagic encephalomyelitis.

6. The use of any of claims 1 to 5, wherein the IGF structural analog is human des(1-3)IGF-I.

7. The use of any of claims 1 to 5, wherein the IGF structural analog is human [Arg3]IGF-I, [Leu24]IGF-I, [Leu60]IGF-I, Long R3IGF-I, des(1-6)IGF-II, [Gly1]IGF-II, [Arg6]IGF-II or [Leu27]IGF-II.

8. The use of any of claims 1 to 7, wherein the medicament is such that the IGF structural analog may be administered in an amount from about 0.0,1 µg/kg/day up to about 4 mg/kg/day.

9. The use of any of claims 1 to 8, wherein the nonintracranial and nonintravertebral column administration is percutaneous, subcutaneous, intramuscular, intraveneous, intraarterial, intradermal, by inhalation, or intranasal.

10. IGF structural analog for use in the nonintracranial and nonintravertebral column administrative treatment or prevention of neuronal damage in the central nervous system, except where the disease is amyotropic lateral sclerosis.

11. The IGF structural analog of claim 10, wherein the IGF structural analog is human [Arg3]IGF-I, [Leu24]IGF-I, [Leu60]IGF-I, Long R3IGF-I, des(1-6)IGF-II, [Gly1]GF-II, [Arg6]IGF-II, [Leu27]IGF-II or human des(1-3)IGF-I.

12. The IGF structural analog for use of claims 10 or 11, wherein the damage is due to a disorder or disease in the central nervous system.

13. The IGF structural analog for use of claims 10 or 11, wherein the damage is due to a tumor or cancer.

14. The IGF structural analog for use of any of claims 10 to 12, wherein the neuronal damage to the central nervous system is due to a disorder or disease in the post-birth brain or spinal cord.

15. The IGF structural analog for use of claim 14, wherein the neuronal damage in the brain is due to hepatic encephalopathy, Pick's Disease, Huntington's Disease, diffuse cerebral sclerosis of Schilder, Alzheimer's Disease, Parkinson's Disease, AIDS-related dementia, senile dementia, stroke, trauma, cortical-basal ganglionic syndromes, progressive dementia, familial dementia with spastic paraparesis, progressive supranuclear palsy, multiple sclerosis, or acute necrotizing hemorrhagic encephalomyelitis.

## Patentansprüche

1. Verwendung eines strukturellen Analogons von IGF zur Herstellung eines Medikaments zur Behandlung des Zentralnervensystems,
wobei das Medikament zur Behandlung oder Prävention von neuronalen Schäden im Zentralnervensystem dient, außer wenn es sich bei der Krankheit um amyotrope Lateralsklerose handelt,
wobei das Medikament zur nichtintrakraniellen und nicht in die Wirbelsäule erfolgenden Verabreichung bestimmt ist.

2. Verwendung gemäß Anspruch 1, wobei die Schäden auf eine Störung oder Erkrankung im Zentralnervensystem zurückzuführen sind.

3. Verwendung gemäß Anspruch 1 oder 2, wobei die Schäden auf einen Tumor oder Krebs zurückzuführen sind.

4. Verwendung gemäß einem der Ansprüche 1 bis 3, wobei die neuronalen Schäden im Zentralnervensystem auf eine Störung oder Erkrankung im postnatalen Gehirn oder Rückenmark zurückzuführen sind.

5. Verwendung gemäß Anspruch 4, wobei die neuronalen Schäden im Gehirn auf hepatische Enzephalopathie, Pick-Krankheit, Chorea Huntington, diffuse zerebrale Sklerose (Schilder), Alzheimer-Krankheit, Parkinson-Krankheit, AIDS-Demenz, senile Demenz, Schlaganfall, Trauma, kortikobasale Degeneration, progressive Demenz, familiäre Demenz mit spastischer Paraparese, progressive supranukleäre Parese, multiple Sklerose oder akute nekrotisierende hämorrhagische Enzephalomyelitis zurückzuführen sind.

6. Verwendung gemäß einem der Ansprüche 1 bis 5, wobei es sich bei dem strukturellen Analogon von IGF um humanes Des(1-3)IGF-I handelt.

7. Verwendung gemäß einem der Ansprüche 1 bis 5, wobei es sich bei dem strukturellen Analogon von IGF um humanes [Arg3]IGF-I, [Leu24]IGF-I, [Leu60]IGF-I, Long-R3-IGF-I, Des(1-6)IGF-II, [Gly1]IGF-II, [Arg6]IGF-II oder [Leu27]IGF-II handelt.

8. Verwendung gemäß einem der Ansprüche 1 bis 7, wobei das Medikament es ermöglicht, das strukturelle Analogon von IGF in einer Menge von etwa 0,01 µg/kg/Tag bis zu etwa 4 mg/kg/Tag zu verabreichen.

9. Verwendung gemäß einem der Ansprüche 1 bis 8, wobei die nichtintrakranielle und nicht in die Wirbelsäule erfolgende Verabreichung perkutan, subkutan, intramuskulär, intravenös, intraarteriell, intradermal, durch Inhalation oder intranasal erfolgt.

10. Strukturelles Analogon von IGF zur Verwendung bei der Behandlung oder Prävention von neuronalen Schäden im Zentralnervensystem durch nichtintrakranielle und nicht in die Wirbelsäule erfolgende Verabreichung, außer wenn es sich bei der Krankheit um amyotrope Lateralsklerose handelt.

11. Strukturelles Analogon von IGF gemäß Anspruch 10, wobei es sich bei dem strukturellen Analogon von IGF um humanes [Arg3]IGF-I, [Leu24]IGF-I, [Leu60]IGF-I, Long-R3-IGF-I, Des(1-6)IGF-II, [Gly1]IGF-II, [Arg6]IGF-II, [Leu27]IGF-II oder humanes Des(1-3)IGF-I handelt.

12. Strukturelles Analogon von IGF zur Verwendung gemäß Anspruch 10 oder 11, wobei die Schäden auf eine Störung oder Erkrankung im Zentralnervensystem zurückzuführen sind.

13. Strukturelles Analogon von IGF zur Verwendung gemäß Anspruch 10 oder 11, wobei die Schäden auf einen Tumor oder Krebs zurückzuführen sind.

14. Strukturelles Analogon von IGF zur Verwendung gemäß einem der Ansprüche 10 bis 12, wobei die neuronalen Schäden im Zentralnervensystem auf eine Störung oder Erkrankung im postnatalen Gehirn oder Rückenmark zurückzuführen sind.

15. Strukturelles Analogon von IGF zur Verwendung gemäß Anspruch 14, wobei die neuronalen Schäden im Gehirn auf hepatische Enzephalopathie, Pick-Krankheit, Chorea Huntington, diffuse zerebrale Sklerose (Schilder), Alzheimer-Krankheit, Parkinson-Krankheit, AIDS-Demenz, senile Demenz, Schlaganfall, Trauma, kortikobasale Degeneration, progressive Demenz, familiäre Demenz mit spastischer Paraparese, progressive supranukleäre Parese, multiple Sklerose oder akute nekrotisierende hämorrhagische Enzephalomyelitis zurückzuführen sind.

## Revendications

1. Utilisation d'un analogue structural d'IGF dans la préparation d'un médicament pour traiter le système nerveux central,
dans laquelle le médicament est destiné à traiter ou prévenir les dommages neuronaux dans le système nerveux central, sauf lorsque la maladie est la sclérose latérale amyotrophique,
dans laquelle le médicament est pour administration non intracrânienne ou non intra-colonne vertébrale.

2. Utilisation de la revendication 1, dans laquelle les dommages sont dus à un trouble ou une maladie dans le système nerveux central.

3. Utilisation de la revendication 1 ou 2, dans laquelle les dommages sont dus à une tumeur ou un cancer.

4. Utilisation de l'une quelconque des revendications 1 à 3, dans laquelle les dommages neuronaux du système nerveux central sont dus à un trouble ou une maladie dans le cerveau ou la moelle épinière après la naissance.

5. Utilisation de la revendication 4, dans laquelle les dommages neuronaux dans le cerveau sont dus à une encéphalopathie hépatique, la maladie de Pick, la maladie de Huntington, la sclérose cérébrale diffuse de Schilder, la maladie d'Alzheimer, la maladie de Parkinson, la démence associée au SIDA, la démence sénile, un accident cérébrovasculaire, un traumatisme, la dégénérescence des noyaux gris centraux corticaux, la démence progressive, la démence familiale avec paraparésie spastique, la paralysie supranucléaire progressive, la sclérose en plaques, ou l'encéphalomyélite hémorragique nécrosante aiguë.

6. Utilisation de l'une quelconque des revendications 1 à 5, dans laquelle l'analogue structural d'IGF est des(1-3)IGF-I humain.

7. Utilisation de l'une quelconque des revendications 1 à 5, dans laquelle l'analogue structural d'IGF est [Arg3]IGF-I, [Leu24]IGF-I, [Leu60]IGF-I, R3-IGF-1 long, des(1-6)IGF-II, [Glyl]IGF-II, [Arg6]IGF-II ou [Leu27]IGF-II humain.

8. Utilisation de l'une quelconque des revendications 1 à 7, dans laquelle le médicament est tel que l'analogue structural d'IGF puisse être administré en une quantité d'environ 0,01 µg/kg/jour à environ 4 mg/kg/jour.

9. Utilisation de l'une quelconque des revendications 1 à 8, dans laquelle l'administration non intracrânienne et non intra-colonne vertébrale est percutanée, sous-cutanée, intramusculaire, intraveineuse, intra-artérielle, intradermique, par inhalation ou intranasale.

10. Analogue structural d'IGF pour utilisation dans le traitement ou la prévention par administration non intracrânienne et non intra-colonne vertébrale de dommages neuronaux dans le système nerveux central, sauf lorsque la maladie est la sclérose latérale amyotrophique.

11. Analogue structural d'IGF de la revendication 10, **caractérisé en ce que** l'analogue structural d'IGF est [Arg3]IGF-I, [Leu24]IGF-I, [Leu60]IGF-I, R3-IGF-I long, des(1-6)IGF-II, [Gly1]IGF-II, [Arg6]IGF-II, [Leu27]IGF-II ou des(1-3)IGF-I humain.

12. Analogue structural d'IGF pour utilisation des revendications 10 ou 11, **caractérisé en ce que** les dommages sont dus à un trouble ou une maladie dans le système nerveux central.

13. Analogue structural d'IGF pour utilisation des revendications 10 ou 11, **caractérisé en ce que** les dommages sont dus à une tumeur ou un cancer.

14. Analogue structural d'IGF pour utilisation de l'une quelconque des revendications 10 à 12, **caractérisé en ce que** les dommages neuronaux du système nerveux central sont dus à un trouble ou une maladie dans le cerveau ou la moelle épinière après la naissance.

15. Analogue structural d'IGF pour utilisation de la revendication 14, **caractérisé en ce que** les dommages neuronaux dans le cerveau sont dus à une encéphalopathie hépatique, la maladie de Pick, la maladie de Huntington, la sclérose cérébrale diffuse de Schilder, la maladie d'Alzheimer, la maladie de Parkinson, la démence associée au SIDA, la démence sénile, un accident cérébrovasculaire, un traumatisme, la dégénérescence des noyaux gris centraux corticaux, la démence progressive, la démence familiale avec paraparésie spastique, la paralysie supranucléaire progressive, la sclérose en plaques, ou l'encéphalomyélite hémorragique nécrosante aiguë.
